# EUROPÄISCHE PATENTSCHRIFT

(11) **EP 3 468 627 B1**
(45) Veröffentlichungstag und Bekanntmachung des Hinweises auf die Patenterteilung: **23.02.2022**
(21) Anmeldenummer: 17727880.1
(22) Anmeldetag: 02.06.2017
(51) Int. Cl.: A61L 27/10, A61L 27/56, C04B 35/48, A61G 3/06, A61G 3/08, B62K 5/023, C04B 35/486

(54) **KNOCHENERSATZMATERIAL AUS ZIRKONDIOXIDKERAMIK**
BONE REPLACEMENT MATERIAL CONSISTING OF ZIRCONIUM DIOXIDE CERAMIC
MATÉRIAU SUBSTITUT OSSEUX EN CÉRAMIQUE AU DIOXYDE DE ZIRCONIUM

(30) Priorität: 09.06.2016 DE 102016110622; 22.06.2016 DE 102016111431
(43) Veröffentlichungstag der Anmeldung: 17.04.2019
(73) Patentinhaber: Gahlert, Michael, 81925 München (DE); Gahlert, Stefan, 70619 Stuttgart (DE)
(72) Erfinder: Gahlert, Michael, 81925 München (DE); Gahlert, Stefan, 70619 Stuttgart (DE)
(74) Vertreter: Witte, Weller & Partner Patentanwälte mbB
(86) Internationale Anmeldenummer: PCT/EP2017/063552
(87) Internationale Veröffentlichungsnummer: WO 2017/211741

(56) Entgegenhaltungen:
- US-A1- 2004 043 051
- US-A1- 2005 100 578
- US-A1- 2011 136 653

## Beschreibung

Die Erfindung betrifft ein Knochenersatzmaterial.

In der zahnärztlichen Implantologie werden kompromittierte anatomische Knochenstrukturen häufig mit zusätzlichen Knochenaufbaumaßnahmen ergänzt. Neben der Möglichkeit, körpereigenen, vitalen Knochen einer Spenderregion in die Empfängerregion zu transferieren (autologer Knochen), gibt es auch die Möglichkeit, ein Knochenersatzmaterial (KEM) zu verwenden.

Es wird dabei zwischen allogenen, alloplastischen und xenogenen KEM unterschieden. Unter allogenem KEM versteht man Knochen von anderen Menschen, der labortechnisch durch Entkalkung oder Gefriertrocknung aufbereitet wurde. Das alloplastische KEM bezeichnet synthetisch hergestelltes KEM wie zum Beispiel Hydroxylapatit, während man xenogenes KEM aus Rinderknochen, Algen oder Korallen herstellt. Bis auf das körpereigene, autologe KEM, welches die Knochenbildung anregt und als osseoinduktiv bezeichnet wird, sind alle anderen KEM osseokonduktiv. Darunter versteht man eine Leitschienenfunktion des KEM, welches ein Einsprossen von ortsständigen Kieferknochen, dem sogenannten Lagergewebe, ermöglichen soll.

Biokompatible KEM sind in der dentalen Implantologie weltweit anerkannt und in zahlreichen Studien evidenzbasiert wissenschaftlich untersucht.

Alloplastische KEM sind im Stand der Technik seit Jahrzehnten in Form diverser anorganischer KEM bekannt. Meist enthalten sie Hydroylapatit und/oder Calciumphosphat (vgl. z.B. DE 100 60 036 C1, EP 0 006 544 A1). Teilweise wird auch Kalziumphosphat verwendet, das zu einer porösen Keramik verarbeitet wird, die auch partikelförmig vorliegen kann (vgl. DE 102 58 773 A1).

Teilweise werden dem anorganischen Knochenersatzmaterial, wie etwa Hydroxlapatit, auch organische Bestandteile beigemischt, wie etwa Kollagen, um die Osseokonduktivität zu verbessern (vgl. z.B. EP 2 826 495 A1).

Grundsätzlich besteht bei anorganischen Knochenersatzmaterialien immer das Problem, dass diese synthetisch hergestellten KEM die Knochenbildung nicht selbst anregen, sondern lediglich eine gewisse Leitschienenfunktion für die Knochenbildung bereitstellen können.

Aus der US 2011/0136653 A1 ist ein Implantat mit einer porösen keramischen Oberflächenschicht bekannt. Allerdings ist dies nicht als Knochenersatzmaterial geeignet.

Aus der US 2004/0043051 A1 ist ferner ein Verbundmaterial bestehend aus einem porösen, inorganischen Material mit Poren bekannt, die mit einem polymeren Material infiltriert sind. Das poröse, inorganische Material besteht aus Calciumphosphat, Hydroxyapatit, MgO, ZrO2, PSZ oder ZrO2-SiO2. Auch dieses Material ist nicht als Knochenersatzmaterial geeignet.

Vor diesem Hintergrund liegt der Erfindung die Aufgabe zu Grunde, ein Knochenersatzmaterial auf anorganischer Basis bereit zu stellen, das sehr gut körperverträglich ist, das eine möglichst gute Osseokonduktivität aufweist und das insbesondere in der dentalen Praxis verwendet werden kann, um in möglichst kurzer Einheilzeit eine dauerhafte Knochenbildung bzw. Osseointegration zu gewährleisten, die insbesondere zum Setzen von Implantaten geeignet ist. Ferner soll ein geeignetes Verfahren zur Herstellung eines solchen Materials angegeben werden

Diese Aufgabe wird durch ein Knochenersatzmaterial gemäß Anspruch 1 und durch ein Verfahren gemäß Anspruch 15 gelöst.

Die Aufgabe der Erfindung wird auf diese Weise vollständig gelöst.

Zirkondioxidkeramik zeichnet sich durch eine exzellente Biokompatibilität und eine vollständige Allergiefreiheit aus. Auch ohne aufwändige Zusatzmaßnahmen, wie eine Beimischung von künstlichen organischen oder tierischen Zusatzmaterialien kann eine gute Osseokonduktivität erreicht werden.

Erfindungsgemäß wird das KEM in Partikelform verwendet, mit einer Partikelgröße im Bereich von 0,1 bis 6 mm, bevorzugt im Bereich von 0,2 bis 4 mm.

Dies erlaubt eine unmittelbare Verwendung in der zahnärztlichen Implantologie, um einen individuellen Knochenaufbau in Defektbereichen zu ermöglichen.

Vorzugsweise ist das KEM makroporös mit einer offenen Porosität.

Es kann sich hierbei etwa um Makroporen mit einem mittleren Porendurchmesser im Bereich von 10 Mikrometer bis 500 Mikrometer, vorzugsweise im Bereich von 50 Mikrometer bis 300 Mikrometer, weiter bevorzugt im Bereich von 60 bis 250 Mikrometer handeln.

Durch eine solche offene Porosität wird die Osseokonduktivität deutlich verbessert. Die untereinander verbundenen Poren erlauben das Einwachsen von Blutzellen und Proteinen und erlauben so eine verbesserte Osseointegration.

Gemäß einem weiteren Merkmal der Erfindung ist das KEM mikroporös, vorzugsweise mit einer offenen Porosität.

Hierbei können die Mikroporen etwa einen mittleren Porendurchmesser von weniger als 10 Mikrometer aufweisen, vorzugsweise von weniger als 1 Mikrometer, vorzugsweise von mindestens 5 Nanometer aufweisen.

Durch eine solche Mikrostruktur ergibt sich eine Kapillarwirkung, die eine Flüssigkeitsaufnahme unterstützt. Auch hierdurch wird also eine Verbesserung der Osseokonduktion unterstützt.

Gemäß einem weiteren Merkmal der Erfindung weist das KEM eine bimodale Porosität mit Makroporen und Mikroporen auf.

Durch die Kombination der offenen Makroporosität mit der offenen Mikroporosität können so beide vorteilhaften Eigenschaften miteinander kombiniert werden. Insgesamt ergibt sich eine weiter verbesserte Osseokonduktion.

In Abhängigkeit von der Partikelgröße ist bei sehr kleinen Partikeln entweder eine massive Ausführung bevorzugt oder aber bei größeren Partikeln gleichfalls eine makroporöse und/oder mikroporöse Ausführung, wie oben beschrieben.

Bei Partikelgrößen ab etwa 1 bis 2 Millimeter Durchmesser ist eine poröse Ausführung bevorzugt, um die Osseointegration zu verbessern.

Bei kleineren Partikelgrößen wird aus Gründen der Stabilität auf eine Makroporosität verzichtet. Allerdings kann eine Mikroporosität zusätzlich die Osseointegration verbessern.

Dagegen wird blockförmiges KEM, das in beliebiger Form hergestellt sein kann, bevorzugt mit offener Makroporosität und ggf. auch offener Mikroporosität verwendet.

Gemäß einem weiteren Merkmal der Erfindung besteht das KEM aus tetragonalem, polykristallinem Zirkondioxid (TZP). Dieses kann etwa mittels Y2O3 stabilisiert sein, beispielsweise durch Zugabe von 3 Gew.-% Y2O3.

Mit TZP werden höchste Festigkeiten erzielt. Grundsätzlich geeignet als KEM ist allerdings auch mit MgO teilstabilisiertes Zirkondioxid (PSZ).

Gemäß einem weiteren Merkmal der Erfindung liegt die Porosität im Bereich von 0,1 bis 90 %, vorzugsweise im Bereich von 1 bis 50 %, weiter bevorzugt im Bereich von 5 bis 30 %.

Mit einer derartigen Porosität wird eine besonders gute Osseokonduktion und Osseointegration erzielt.

Soweit nicht dentale Anwendungen betroffen sind, etwa bei der Rekonstruktion von diversen Knochenfrakturen, kann das KEM auch in speziellen Formen hergestellt werden, die ggf. auch patentientenspezifisch angepasst sein können.

Gemäß einem weiteren Merkmal der Erfindung weist das KEM eine geätzte Oberfläche auf.

Durch eine Ätzbehandlung, etwa mittels Flusssäure, der äußeren Oberfläche und der inneren Oberflächen infolge der offenen Porosität wird die Osseoinduktion weiter verbessert.

Erfindungsgemäß kann ein makroporöser Körper mit einer offenen Porosität erzeugt werden. Da sich die Kurzfasern bzw. die dreidimensionale Struktur oder das Pulver aus organischem Material beim Aufheizen vollständig zersetzen, kann durch die so frei werdenden Gase eine offene Porosität erzeugt werden. Gesteuert wird dies insbesondere durch den Anteil der zugesetzten Fasern, sowie deren Durchmesser und Länge, bzw. Gestaltung der dreidimensionalen Struktur, die Partikelgröße des organischen Pulvers, die Partikelgröße des Zirkondioxidpulvers und das verwendete Temperaturprogramm.

Eine bimodale Porosität kann hierbei erzeugt werden, indem der Sintervorgang gesteuert beendet wird, bevor die beim Sintern normalerweise anfangs vorhandenen Mikroporen vollständig auswachsen. Alternativ können auch organische Zusätze mit bimodaler Größenverteilung zugesetzt werden.

Soweit eine dreidimensionale Struktur etwa in Form eines Gewebes oder Gewirkes verwendet wird, so kann diese in geeigneter Weise dimensioniert werden, um eine möglichst homogene offene Porosität beim späteren Brennvorgang zu erzeugen. Auch eine Erzeugung einer Porosität mit Textur ist möglich.

Zur Herstellung einer homogenen Mischung der Komponenten ist ein Pelletieren in einem Pelletiergefäß, also auf einem drehbar angetriebenen Pelletierteller oder in einer drehbar angetriebenen Trommel besonders geeignet.

Gemäß einem weiteren Merkmal der Erfindung erfolgt die Formgebung durch Pressen, vorzugsweise uniaxiales oder isostatisches Pressen, durch Schlickergießen oder Schleudergießen mit anschließenden Trocknen, oder durch ein anders pulvertechnologisches Verfahren.

Auch hierbei werden vorzugsweise organische Zusätze, insbesondere Kurzfasern oder dreidimensionale Strukturen aus Kunststoff, mit geeignetem Durchmesser und geeigneter Länge zugesetzt, die sich beim späteren Sintern zersetzen, um so eine makroporöse Struktur zu erzeugen.

Gemäß einem weiteren Merkmal der Erfindung wird ein poröses KEM aus einer Zirkondioxidkeramik hergestellt, indem ein offenporiger Kunststoffschaum unter Entlüftung in einen Schlicker mit Zirkondioxidpulver getaucht wird, anschließend aus dem Schlicker entfernt, getrocknet und gesintert wird.

Auch auf diese Weise lässt sich ein makroporöses KEM aus Zirkondioxid herstellen. Dieses Verfahren ist im Zusammenhang mit der Herstellung von porösen Keramikkörpern als sog. Replikaverfahren bekannt.

Gemäß einem weiteren Merkmal der Erfindung wird das erhaltene Material nach dem Sintern zu Partikeln gemahlen.

Gemäß einem weiteren Merkmal der Erfindung wird mikroskaliges Zirkondioxidpulver mit einer mittleren spezifischen Oberfläche im Bereich von 5 bis 100 m²/g verwendet.

Gemäß einem weiteren Merkmal der Erfindung erfolgt das Sintern im Bereich von 850 °C bis 1750 °C, vorzugsweise im Bereich von 850 °C bis 1550 °C, weiter bevorzugt im Bereich von 900 °C bis 1350 °C.

Die verwendete Sintertemperatur ist insbesondere von der Pulverbeschaffenheit des Zirkondioxidpulvers, insbesondere von der mittleren spezifischen Oberfläche oder der mittleren Partikelgröße abhängig. Je größer die mittlere spezifische Oberfläche, um so geringer ist in der Regel die Sintertemperatur.

Mit Pulvern im Bereich von etwa 20 bis 50 m²/g liegen die Sintertemperaturen meist im Bereich von etwa 1250 °C bis 1350 °C.

Soweit ein Vorsintern zu einem Grünkörper erfolgt, der im Grünzustand mechanisch bearbeitet werden kann, um eine bestimmte Form und Größe herzustellen, liegt die Vorsintertemperatur etwa 100 bis 500 K unterhalb der Temperatur zum Fertigsintern.

### BEISPIELE

### Beispiel 1

Kommerziell erhältliches TZP-Pulver (mit 3 Gew.-% Y₂O₃ stabilisiert) mit einer mittleren spezifischen Oberfläche von 50 m²/g wird mit 20 Gew.-% Kurzfasern aus Polyvinychlorid (PVC) mit einem mittleren Durchmesser von etwa 50 Mikrometer und einer mittleren Länge von 500 Mikrometer unter Zusatz eines Bindemittels (1 Gew.-% Isopropanol) gemischt, so dass die Kurzfasern statistisch verteilt sind. Danach erfolgt ein uniaxiales Pressen unter Verwendung einer geeigneten Stahlmatrize (Druck von beispielsweise 20 bis 50 bar).

Anschließend erfolgt eine Aufheizung auf etwa 1350 °C mit etwa 50 K/min und ein Halten bei 1350 °C für etwa 30 bis 120 Minuten, danach Abkühlung durch Abschalten.

Auf diese Weise wird ein makroporöser Block aus TZP-Keramik mit einer offenen Porosität erhalten. Das so erhaltene Material wird mittels einer Kugelmühle zu porösen Partikeln vermahlen und dann für eine gewünschte Partikelgrößenverteilung entsprechend ausgesiebt.

Anstelle einer uniaxialen Pressung kann das Pulvergemisch auch isostatisch gepresst werden.

### Beispiel 2

Zur Herstellung von pulverförmigem KEM, das nicht porös ist, wird kommerziell erhältliches Zirkondioxid zur gewünschten Partikelgrößenverteilung gemahlen und anschließend ausgesiebt. Dies ist insbesondere für kleinere Partikelgrößen etwa kleiner als 1 bis 2 Millimeter Durchmesser, von Interesse.

### Beispiel 3

Das Material gemäß Beispiel 1 wird nicht vermahlen, sondern unmittelbar in Blockform verwendet. Es kann hierzu durch mechanische Bearbeitung mit Diamantwerkzeugen in die gewünschte Größe und/oder Form gebracht werden.

### Beispiel 4

Kommerziell erhältliches TZP-Pulver (mit 3 Gew.-% Y₂O₃ stabilisiert) mit einer mittleren spezifischen Oberfläche von 50 m²/g wird mit 5 Gew.-% mit 10 Gew.-% Kurzfasern aus Polyvinychlorid (PVC) mit einem mittleren Durchmesser von etwa 50 Mikrometer und einer mittleren Länge von 500 Mikrometer unter Zusatz eines Bindemittels (1 Gew.-% Isopropanol) gemischt und dann isostatisch gepresst (Druck von beispielsweise 500 bis 1000 bar).

Anschließend erfolgt eine Aufheizung auf etwa 900 °C mit etwa 50 K/min und ein Halten für etwa 15 bis 30 Minuten, danach Abkühlung durch Abschalten. Es wird ein makroporöser Grünkörper erhalten, der im Grünzustand mechanisch bearbeitet werden kann, etwa mittels einer automatisch gesteuerten Fräseinrichtung. Dabei wird das Schrumpfungsmaß für den späteren Sinterprozess berücksichtigt.

Schließlich erfolgt ein Fertigsintern bei etwa 1300 °C (Aufheizen mit etwa 50 K/min und dann Halten bei 1300 °C etwa 15 bis 60 Minuten). Es ergibt sich ein makroporöser Zirkondioxidkörper mit offener Porosität.

### Beispiel 5

Ein offenporiger Kunststoffschaum wird in einen Schlicker mit Zirkonoxidpulver gemäß Beispiel 1 getaucht, mit dem Schlicker getränkt, anschließend getrocknet (60 Minuten bei 90 °C), dann gemäß Beispiel 1 gesintert. Es ergibt sich eine offenporige Zirkonoxidkeramik.

### Beispiel 6

Als Nachbehandlung wird bei den Beispielen 1 bis 5 eine Ätzbehandlung durch Eintauchen in Flusssäure durchgeführt, in dem das fertig gesinterte und ggf. gemahlene KEM beispielsweise in 40%-ige Flusssäure getaucht wird und 5 bis 60 Minuten geätzt wird, vorzugsweise bei erhöhter Temperatur von z.B. 60 bis 70 °C.

## Patentansprüche

1. Knochenersatzmaterial zur Verwendung als Knochenaufbaumaterial in der zahnärztlichen Implantologie, bestehend aus einer Zirkondioxidkeramik in Partikelform mit einer Partikelgröße im Bereich von 0,1 bis 6 mm, vorzugsweise im Bereich von 0,2 bis 4 mm, vorzugsweise mit einer durch eine Ätzbehandlung aufgerauten Oberfläche.

2. Knochenersatzmaterial nach Anspruch 1, das makroporös mit einer offenen Porosität ist und/oder das mikroporös, vorzugsweise mit einer offenen Porosität ist.

3. Knochenersatzmaterial nach Anspruch 2, das Makroporen mit einem mittleren Porendurchmesser im Bereich von 10 Mikrometer bis 500 Mikrometer, vorzugsweise im Bereich von 50 Mikrometer bis 300 Mikrometer, weiter bevorzugt im Bereich von 60 bis 250 Mikrometer aufweist.

4. Knochenersatzmaterial nach Anspruch 2 oder 3, das Mikroporen mit einem mittleren Porendurchmesser von weniger als 10 Mikrometer aufweist, vorzugsweise von weniger als 1 Mikrometer, vorzugsweise von mindestens 5 Nanometer aufweist.

5. Knochenersatzmaterial nach einem der vorhergehenden Ansprüche, mit einer bimodalen Porosität mit Makroporen und Mikroporen.

6. Knochenersatzmaterial nach einem der vorhergehenden Ansprüche, bestehend aus tetragonalem, polykristallinem Zirkondioxid (TZP).

7. Knochenersatzmaterial nach einem der vorhergehenden Ansprüche, bei dem die Porosität im Bereich von 0,1 bis 90 % liegt, vorzugsweise im Bereich von 1 bis 50 %, weiter bevorzugt im Bereich von 5 bis 30 %.

8. Knochenersatzmaterial nach einem der vorhergehenden Ansprüche, in Blockform, vorzugsweise makroporös mit offener Porosität.

9. Verfahren zum Herstellen eines porösen Knochenersatzmaterials aus einer Zirkondioxidkeramik zur Verwendung als Knochenaufbaumaterial in der zahnärztlichen Implantologie in Partikelform, bei dem Zirkondioxidpulver mit organischen Zusätzen, insbesondere in Form von Kurzfasern, dreidimensionalen Strukturen, etwa in Form eines Gewebes oder Gewirkes, und/oder in Form von Pulver, vorzugsweise aus Kunststoff, versetzt wird, vorzugsweise unter Zugabe von Bindemitteln, zu einem Precurser-Körper geformt wird, und anschließend gesintert wird, und wobei das erhaltene Material nach dem Sintern zu Partikeln mit einer Partikelgröße von 0,1 bis 6 mm gemahlen wird.

10. Verfahren nach Anspruch 9, bei dem Kurzfasern mit einem mittleren Durchmesser von im Bereich von 10 Mikrometer bis 500 Mikrometer, vorzugsweise im Bereich von 50 Mikrometer bis 300 Mikrometer, weiter bevorzugt im Bereich von 60 bis 250 Mikrometer verwendet werden, wobei vorzugsweise Kurzfasern mit einer mittleren Länge von 10 Mikrometer bis 20 Millimeter, vorzugsweise von 50 Mikrometer bis 2000 Mikrometer, besonders bevorzugt von 100 Mikrometer bis 1000 Mikrometer verwendet werden, wobei vorzugsweise Kurzfasern mit bimodaler Durchmesserverteilung zugesetzt werden.

11. Verfahren nach einem der Ansprüche 9 bis 10, bei dem eine dreidimensionale Struktur aus Fasern mit einem mittleren Durchmesser im Bereich von 10 Mikrometer bis 500 Mikrometer, vorzugsweise im Bereich von 50 Mikrometer bis 300 Mikrometer, weiter bevorzugt im Bereich von 60 bis 250 Mikrometer, verwendet wird.

12. Verfahren nach einem der Ansprüche 9 bis 11, bei dem zusätzlich zu den Kurzfasern oder der dreidimensionalen Struktur ein organisches Pulver, vorzugsweise mit bimodaler Partikelgrößenverteilung zugesetzt wird.

13. Verfahren nach einem der Ansprüche 9 bis 12, bei dem die Mischung der Komponenten durch Pelletieren in einem Pelletiergefäß zu einem homogenen Agglomerat erfolgt.

14. Verfahren nach einem der Ansprüche 9 bis 13, bei dem die Formgebung durch Pressen, vorzugsweise uniaxiales oder isostatisches Pressen, durch Schlickergießen oder Schleudergießen mit anschließender Trocknung erfolgt.

15. Verfahren nach einem der Ansprüche 9 bis 14, bei dem das Knochenersatzmaterial nach dem Sintern geätzt wird, insbesondere in eine Ätzlösung wie etwa Flusssäure, getaucht wird.

## Claims

1. Bone substitute material for use as bone augmentation material in dental implantology, consisting of a zirconium dioxide ceramic in particle form with a particle size in the range from 0.1 to 6 mm, preferably in the range from 0.2 to 4 mm, preferably with a surface roughened by an etching treatment.

2. Bone substitute material according to claim 1, which is macroporous with an open porosity and/or which is microporous, preferably with an open porosity.

3. Bone substitute material according to claim 2, which has macropores with an average pore diameter in the range of 10 micrometers to 500 micrometers, preferably in the range of 50 micrometers to 300 micrometers, more preferably in the range of 60 to 250 micrometers.

4. Bone substitute material according to claim 2 or 3, which has micropores with a mean pore diameter of less than 10 micrometers, preferably of less than 1 micrometer, preferably of at least 5 nanometers.

5. Bone substitute material according to any one of the preceding claims, having a bimodal porosity with macropores and micropores.

6. Bone substitute material according to any one of the preceding claims, consisting of tetragonal polycrystalline zirconia (TZP).

7. Bone substitute material according to any one of the preceding claims, wherein the porosity is in the range of 0.1 to 90%, preferably in the range of 1 to 50%, more preferably in the range of 5 to 30%.

8. Bone substitute material according to any of the preceding claims, in block form, preferably macroporous with open porosity.

9. Method for producing a porous bone substitute material from a zirconium dioxide ceramic for use as a bone augmentation material in dental implantology in particle form, in which zirconium dioxide powder with organic additives, in particular in the form of short fibers, three-dimensional structures, for example in the form of a woven or knitted fabric, and/or in the form of powder, preferably of plastic, preferably with the addition of binding agents, is formed into a precursor body, and is subsequently sintered, and wherein the material obtained, after sintering, is ground to particles with a particle size of 0.1 to 6 mm.

10. Method according to claim 9, wherein short fibers having an average diameter of in the range of 10 microns to 500 microns, preferably in the range of 50 microns to 300 microns, more preferably in the range of 60 to 250 microns are used, wherein preferably short fibers having an average length of from 10 micrometers to 20 millimeters, preferably from 50 micrometers to 2000 micrometers, more preferably from 100 micrometers to 1000 micrometers are used, wherein preferably short fibers having a bimodal diameter distribution are added.

11. Method according to any one of claims 9 to 10, wherein a three-dimensional structure of fibers having an average diameter in the range of from 10 micrometers to 500 micrometers, preferably in the range of from 50 micrometers to 300 micrometers, more preferably in the range of from 60 to 250 micrometers, is used.

12. Method according to any one of claims 9 to 11, wherein, in addition to the short fibers or the three-dimensional structure, an organic powder, preferably with bimodal particle size distribution, is added.

13. Method according to any one of claims 9 to 12, in which the mixing of the components is carried out by pelletizing in a pelletizing vessel to form a homogeneous agglomerate.

14. Method according to any one of claims 9 to 13, in which the shaping is carried out by pressing, preferably uniaxial or isostatic pressing, by slip energy casting or centrifugal casting with subsequent drying.

15. Method according to any one of claims 9 to 14, wherein the bone substitute material is etched after sintering, in particular immersed in an etching solution such as hydrofluoric acid.

## Revendications

1. Matériau de substitution osseuse destiné à être utilisé comme matériau d'augmentation osseuse en implantologie dentaire, constitué d'une céramique de zircone sous forme de particules ayant une taille de particule comprise entre 0,1 et 6 mm, de préférence entre 0,2 et 4 mm, de préférence avec une surface rendue rugueuse par un traitement de gravure.

2. Matériau de substitution osseuse selon la revendication 1, qui est macroporeux avec une porosité ouverte et/ou qui est microporeux, de préférence avec une porosité ouverte.

3. Matériau de substitution osseuse selon la revendication 2, ayant des macropores avec un diamètre de pore moyen dans la gamme de 10 microns à 500 microns, de préférence dans la gamme de 50 microns à 300 microns, plus préférablement dans la gamme de 60 à 250 microns.

4. Matériau de substitution osseuse selon la revendication 2 ou 3, qui présente des micropores avec un diamètre moyen de pore inférieur à 10 micromètres, de préférence inférieur à 1 micromètre, de préférence d'au moins 5 nanomètres.

5. Matériau de substitution osseuse selon l'une quelconque des revendications précédentes, présentant une porosité bimodale avec des macropores et des micropores.

6. Matériau de substitution osseuse selon l'une quelconque des revendications précédentes, comprenant de la zircone polycristalline tétragonale (TZP).

7. Matériau de substitution osseuse selon l'une quelconque des revendications précédentes, dans lequel la porosité est comprise entre 0,1 et 90%, de préférence entre 1 et 50%, plus préférablement entre 5 et 30%.

8. Matériau de substitution osseuse selon l'une quelconque des revendications précédentes, sous forme de bloc, de préférence macroporeux à porosité ouverte.

9. Procédé de production d'un matériau poreux de substitution osseuse à partir d'une céramique de zircone, destiné à être utilisé comme matériau d'augmentation osseuse en implantologie dentaire sous forme de particules, dans lequel la poudre de zircone avec des additifs organiques, en particulier sous forme de fibres courtes, des structures tridimensionnelles, par exemple sous forme d'un tissu ou d'un tricot, et/ou sous forme de poudre, de préférence en matière plastique, est ajouté, de préférence avec l'ajout de liants, est formé en un corps précurseur, et est ensuite fritté, et dans lequel le matériau obtenu, après frittage, est broyé en particules d'une taille de 0,1 à 6 mm.

10. Procédé selon la revendication 9, dans lequel on utilise des fibres courtes ayant un diamètre moyen de l'ordre de 10 microns à 500 microns, de préférence de l'ordre de 50 microns à 300 microns, plus préférablement de l'ordre de 60 à 250 microns, dans lequel on utilise de préférence des fibres courtes ayant une longueur moyenne de 10 micromètres à 20 millimètres, de préférence de 50 micromètres à 2000 micromètres, plus préférablement de 100 micromètres à 1000 micromètres, dans lequel on ajoute de préférence des fibres courtes ayant une distribution de diamètre bimodale.

11. Procédé selon l'une quelconque des revendications 9 à 10, dans lequel on utilise une structure tridimensionnelle de fibres ayant un diamètre moyen compris entre 10 micromètres et 500 micromètres, de préférence compris entre 50 micromètres et 300 micromètres, plus préférablement compris entre 60 et 250 micromètres.

12. Procédé selon l'une quelconque des revendications 9 à 11, dans lequel, en plus des fibres courtes ou de la structure tridimensionnelle, on ajoute une poudre organique, de préférence à distribution granulométrique bimodale.

13. Procédé selon l'une quelconque des revendications 9 à 12, dans lequel le mélange des compo- nents est réalisé par granulation dans un récipient de granulation pour former un agglomérat homogène.

14. Procédé selon l'une quelconque des revendications 9 à 13, dans lequel la mise en forme est effectuée par pressage, de préférence par pressage uniaxial ou isostatique, par coulage à énergie de glissement ou par coulage centrifuge avec séchage ultérieur.

15. Procédé selon l'une quelconque des revendications 9 à 14, dans lequel le matériau de substitution osseuse est gravé après frittage, en particulier immergé dans une solution de gravure telle que l'acide fluorhydrique.
